# EUROPEAN PATENT APPLICATION

(11) **EP 4 226 932 A1**
(43) Date of publication of application: **16.08.2023**
(21) Application number: 21822081.2
(22) Date of filing: 01.06.2021
(51) Int. Cl.: A61K 38/08, A61P 1/02, C07K 7/06

(54) **PHARMACEUTICAL COMPOSITION FOR PREVENTING OR TREATING PERIODONTAL DISEASE OR LUXATED TEETH**

(30) Priority: 09.06.2020 KR 20200069444
(71) Applicant: Hysensbio, Gyeonggi-do 13814 (KR)
(72) Inventor: PARK, Joo Hwang, Incheon 21986 (KR); LEE, Ji Hyun, Seoul 03088 (KR); LEE, Dong Seol, Seoul 03088 (KR)
(74) Representative: Mewburn Ellis LLP
(86) International application number: PCT/KR2021/006812
(87) International publication number: WO 2021/251673

(57) **Abstract**

The present invention provides a pharmaceutical composition for preventing or treating periodontal disease or a dislocated traumatic tooth, comprising a peptide of a specific amino acid sequence and a composition for regenerating periodontium.

## Description

### [Technical field]

The present invention relates to a pharmaceutical composition for preventing or treating a periodontal disease or a dislocated traumatic tooth, and a composition for regenerating periodontium.

### [Background Art]

Periodontium is a generic term for tissues surrounding teeth. The periodontium consists of alveolar bone that supports teeth within a maxilla (jawbone), a periodontal ligament that connects alveolar bone and teeth within the maxilla, cementum including periodontal ligament fibers, and gingiva, which is a soft tissue covering the alveolar bone. Gingival fibroblast and periodontal ligament fibroblast are major cellular components of a gingival soft tissue-bearing connective tissue, and play a role in forming and maintaining an extracellular matrix. Among these, the gingival fibroblast is mainly involved in maintaining a gingival connective tissue, whereas periodontal ligament fibroblast is known to be involved in the restoration and regeneration of an adjacent alveolar bone and cementum in vivo as well as forming the periodontal ligament as an unique function thereof.

Periodontal ligament refers to a fibrous connective tissue that connects cementum of tooth root (root part) and alveolar bone. Both ends of the periodontal ligament are embedded in the root cementum and alveolar bone, and a periodontal ligament fiber bundle embedded in the alveolar bone or cementum is called Sharpey's fiber. A function of the periodontal ligament is not only to hold teeth and to hold structural properties of hard tissues but also to resist the impact of occlusal pressure and to protect soft tissues such as blood vessels and nerves from damage. Another important function is to supply nutrients and function as sensory receptors, and innervation of the periodontal ligament transmits proprioceptive sensations, tactile sensations, and pain sensations through a trigeminal nerve pathway to sense and regulate external pressure applied to individual teeth, and to play an important role in neuromuscular mechanisms regulating masticatory musculature. The periodontal ligament contains many fibroblasts, and these cells not only form the periodontal ligament as a unique function thereof, but also have functions of being involved in a formation and resorption of cementum and an alveolar bone to induce physiological tooth migration and allowing periodontium to adapt to an occlusal pressure and repairing damage.

Periodontal disease is classified into gingivitis limited to gingiva and periodontitis, in which inflammation spreads to an alveolar bone surrounding the tooth root, and is not a disease that damages teeth itself, but a disease that causes inflammation in the periodontium that supports a tooth. When periodontal disease occurs, it clinically causes tooth loss due to gingival bleeding and swelling, formation of the periodontal pocket, and destruction of alveolar bone, and causes of the periodontal disease include local and systemic factors. Two known local causative factors are dental plaque, tartar, and other local causes and trauma from occlusion. As a pathogenesis mechanism, when a dental plaque is mechanically accumulated in the periodontal pocket, it becomes a habitat for surrounding bacteria, this inhabitation gradually shifts from aerobic, aerobic, gram-positive bacteria to anaerobic gram-negative bacteria and proliferates to depths in the periodontal pocket, and these anaerobic gram-negative bacteria proliferate and migrate to depths of the periodontal pocket. At this time, a toxin and all products of proliferated anaerobic gram-negative bacteria directly destroy tissues or stimulate an immune system to induce inflammation along with the destruction of periodontium through various actions from a stimulated immune system. As a defense mechanism against this, the function and immune response of polymorphonuclear leukocytes act as systemic factors. However, it is known that an antibacterial and bacteriostatic action against anaerobic gram-negative bacteria, which are original factors, the removal or destruction of toxic products of these bacteria, and the restoration of lost periodontium are important factors for the prevention and treatment of the periodontal disease.

The basic treatment of periodontal disease includes a tartar removal (scaling) procedure to remove dental plaque and tartar that cause the periodontal disease, and similarly, root planarization is a procedure that removes dental plaque and tartar and removes inflammation-causing factors embedded in the cementum of a tooth root surface. When inflammation is severe due to periodontal disease, it is accompanied by alveolar bone damage, and bone grafting through a regenerative periodontal surgery can be performed when the alveolar bone is severely absorbed. As a type of bone to be transplanted, the patient's own bone (autogenous bone) or xenogeneic or synthetic bone can be used.

Therefore, in order to treat periodontal disease, it is considered that not only the causative bacteria of the periodontal disease should be removed, but also the process of regenerating periodontium should be performed together.

Currently, bone grafting, tissue-guided regeneration, and treatment using various growth factors are being implemented as treatment methods to regenerate a destroyed periodontium. Also, a tooth replantation method, which focuses on the regeneration of periodontal ligament cells and the formation of new cementum, is also being implemented.

In this regard, research is being conducted to develop a method for effectively regenerating the periodontium. For example, Korean Patent Registration No. 1179476 discloses the promyelocytic leukemia zinc finger (PLZF) gene, which is highly expressed in a mineralization process of human periodontal ligament (hPDL) cells, Fk506 binding protein 5 (FKBP5) gene, serum amyloid A1 (SAA1) gene, fatty acid binding protein 4 (FABP4) gene, family A (FAM107A) gene with sequence similarity, CORIN gene, ras-associated C3 botulinum toxin substrate 3 (RAC3) gene or prolactin-derived protein (PIP) gene, lowly expressed FNDC1, PTGS2, RSAD2, NPTX1, VCAM1, MX1, IFIT1, CLDN1 or WISP2 genes during mineralization of human periodontal ligament (hPDL) cells. In addition, Korean Patent Registration No. 101788916 discloses a pharmaceutical composition for treating periodontal disease, including an ameloblast culture solution.

Meanwhile, when a traumatic force is applied to a tooth or periodontium, various destruction occurs in the tooth and periodontium according to the difference in direction and size. Dental trauma can be broadly divided into fracture trauma and dislocation trauma. The dislocation trauma refers to damage to the periodontal ligament, and can be subdivided into concussive subdislocation, orbital dislocation, lateral dislocation, complete dislocation, and intrusion, and occur more often in children aged 8 to 12 years. A success of replanting a completely dislocated tooth depends on the regeneration of the periodontal ligament attached to the completely dislocated tooth or the periodontal ligament remaining in the alveolar fossa from which the tooth is missing. When periodontal ligament regeneration does not occur, the cementum and dentin of the tooth root are absorbed, and ankylosis occurs in which this region is replaced by alveolar bone. Therefore, an ultimate object of periodontal treatment is to prevent the progression of the periodontal disease and regenerate a destroyed periodontium, including gingiva, periodontal ligament, alveolar bone, and cementum to restore it to its original state, but a method that can directly regenerate or differentiate the periodontal ligament has not been reported yet.

Under this background, the present inventors made intensive research efforts to develop a method to regenerate periodontium (periodontal ligament, cementum, and alveolar bone) damaged due to the periodontal disease, and as a result, by inducing differentiation of periodontal ligament cells, a new use of the peptide that can not only treat damaged periodontal ligament of the periodontal disease or the dislocated traumatic tooth but also regenerate the damaged cementum and alveolar bone was confirmed and completed the present invention.

### [Disclosure]

### [Technical Problem]

An object of the present invention is to provide a pharmaceutical composition for preventing or treating a periodontal disease or a dislocated traumatic tooth.

Another object of the present invention is to provide a composition for regenerating periodontium.

Another object of the present invention is to provide a composition for promoting the expression of any one or more genes of BSP, DMP1, CAP, COL3, and Periostin.

Another object of the present invention is to provide a method for treating the periodontal disease or the dislocated traumatic tooth in an individual, the method includes administering an effective amount of a peptide or a pharmaceutical acceptable salt thereof to an individual in need of treatment of the periodontal disease or the dislocated traumatic tooth.

Another object of the present invention is to provide a use of a peptide or a pharmaceutically acceptable salt thereof in the manufacture of a medicament for preventing or treating periodontal disease or the dislocated traumatic tooth.

The objects of the present invention are not limited to those mentioned above, and further objects which are not mentioned will be apparent to those skilled in the art from the following description.

### [Technical Solution]

One aspect of the present invention for solving the above technical problem is to provide a pharmaceutical composition for preventing or treating a periodontal disease or a dislocated traumatic tooth, comprising a peptide or a pharmaceutically acceptable salt thereof comprising an amino acid sequence of the following general formula 1:

K-Y-K-Q-X5-X6-X7-X8-Y-K (General formula 1)

in the general formula 1,
X5 to X7 are each independently arginine (R) or lysine (K);
X8 is asparagine (N) or serine (S).

As long as the peptide can exhibit a preventive or therapeutic effect for the periodontal disease or the dislocated traumatic tooth, a variant peptide having the amino acid sequence constituting the peptide and a sequence different from one or more amino acid residues are also included within the scope of the peptides provided in the present invention.

Throughout the present specification, conventional one-letter and three-letter codes for naturally occurring amino acids constituting peptides are used. Also, amino acids referred to by abbreviation herein are described according to the IUPAC-IUB nomenclature.

| | |
|---|---|
| Alanine: Ala, A | Arginine: Arg, R |
| Asparagine: Asn, N | Aspartic Acid: Asp, D |
| Cysteine: Cys, C | Glutamic Acid: Glu, E |
| Glutamine: Gln, Q | Glycine: Gly, G |
| Histidine: His, H | Isoleucine: Ile, I |
| Leucine: Leu, L | Lysine: Lys, K |
| Methionine: Met, M | Phenylalanine: Phe, F |
| Proline: Pro, P | Serine: Ser, S |
| Threonine: Thr, T | Tryptophan: Trp, W |
| Tyrosine: Tyr, Y | Valine: Val, V |

The present inventors have completed the present invention by confirming that the peptide can treat periodontal disease or dislocated traumatic tooth by promoting the regeneration of periodontal ligament or differentiation into osteoblast and cementoblast, and regeneration of alveolar bone and cementum.

It was confirmed that the peptide increased the expression of BSP, DMP1, and CAP, which are marker genes for osteoblast and cementoblast differentiation, and increased the expression of periostin and COL3, which are marker genes for periodontal ligament differentiation in human periodontal ligament cells. In addition, it was confirmed that the peptide increases the expression of BSP and DMP1, marker genes for osteoblast and cementoblast differentiation, in human mesenchymal stem cells.

In addition, as a result of periodontium damage animal model experiment, it was confirmed that the peptide regenerated alveolar bone, cementum and periodontal ligament. Specifically, it was confirmed that the peptide of the present invention has the effect of regenerating alveolar bone and forming a new cementum-like tissue and a new periodontal ligament-like tissue, and the new periodontal ligament formed by the peptide of the present invention is incorporated into the newly formed alveolar bone and cementum.

In one embodiment, the peptide may be one in which a conservative substitution has occurred in one or more amino acids.

"Conservative substitution" means substituting an amino acid for another amino acid having similar structural and/or chemical properties. The peptides may have, for example, one or more conservative substitutions while still retaining the same or similar biological activity. Such amino acid substitutions may generally occur based on similarity in the polarity, charge, solubility, hydrophobicity, hydrophilicity and/or amphipathic nature of the residues. For example, positively charged (basic) amino acids include arginine, lysine, and histidine; negatively charged (acidic) amino acids include glutamic acid and aspartic acid; aromatic amino acids include phenylalanine, tryptophan, and tyrosine, and hydrophobic amino acids include alanine, valine, isoleucine, leucine, methionine, phenylalanine, tyrosine, and tryptophan. In addition, amino acids can be classified into amino acids having an electrically charged side chain and amino acids having an uncharged side chain, and amino acids having an electrically charged side chain include aspartic acid, glutamic acid, lysine, arginine, and histidine, amino acids including having an uncharged side chain can be classified into nonpolar amino acids or polar amino acids again, and nonpolar amino acids include glycine, alanine, valine, leucine, isoleucine, methionine, and proline, and polar amino acids include serine, threonine, cysteine, asparagine, and glutamine. Conservative substitutions with amino acids having similar properties as described above can be expected to exhibit the same or similar activity.

As such, even when the acidic amino acids, basic amino acids or aromatic amino acids constituting the peptide of the present invention is substituted with another acidic amino acids, basic amino acids, neutral amino acids or aromatic amino acids, the effect of the peptide provided in the present invention can be exhibited as it is, since the effects of the peptide provided in the present invention can be exhibited as they are, it is obvious that a variant peptide having the amino acid sequence constituting the peptide of the present invention and a sequence different from one or more amino acid residues are also included in the scope of the peptides provided in the present invention.

In addition, even when the peptide of the present invention has a form in which any amino acid is added to N-terminus or C-terminus thereof, an effect of the peptide provided in the present invention can be shown as it is, so that it falls within the scope of the peptide provided in the present invention. As an example, it may be in a form in which 1 to 300 amino acids are added to an N-terminus or C-terminus of the peptide, as another example, it may be in a form in which 1 to 100 amino acids are added to the N-terminus or C-terminus of the peptide, and as another example, it may be in a form in which 1 to 24 amino acids are added to the N-terminus or C-terminus of the peptide.

In one embodiment, the peptide includes an amino acid sequence of any one of SEQ ID NOs: 1 to 16.

In another embodiment, the peptide consists essentially of an amino acid sequence of any one of SEQ ID NOs: 1 to 16, or the peptide consists of an amino acid sequence of any one of SEQ ID NOs: 1 to 16.

Even when it is described herein as a 'peptide consisting of a specific SEQ ID NO', when it has the same or corresponding activity as the peptide consisting of an amino acid sequence of the corresponding SEQ ID NO, addition of meaningless sequences before and after the amino acid sequence of the corresponding SEQ ID NO, or mutations that may occur naturally, or silent mutations thereof are not excluded, and even when such sequence addition or mutation is included, it is evident that it falls within the scope of the present invention. That is, even when there is a difference in some sequences, it may fall within the scope of the present invention when it exhibits homology at a certain level or more and exhibits the same or similar activity. Specifically, the peptide of the present invention may include, but is not limited to, an amino acid sequence having 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% or more homology or identity.

"Homology" or "identity" refers to the degree to which two given amino acid sequences or base sequences are related to each other and may be expressed as a percentage. The terms "homology" and "identity" may often be used interchangeably.

Whether any two peptide sequences have homology, similarity or identity can be determined, for example, by Pearson et al (1988) [Proc. Natl. Acad. Sci. USA 85]: 2444, using a known computer algorithm such as the "FASTA" program. In addition, as performed in the Needleman program of the EMBOSS package (EMBOSS: The European Molecular Biology Open Software Suite, Rice et al., 2000, Trends Genet. 16: 276-277) (version 5.0.0 or later), Needleman-Wunsch algorithm (Needleman and Wunsch, 1970, J. Mol. Biol. 48: 443-453) can be used. GCG program package (Devereux, J., et al, Nucleic Acids Research 12: 387 (1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073) may be included. For example, BLAST or ClustalW from the National Center for Biotechnology Information Database can be used to determine homology, similarity, or identity.

The homology, similarity or identity of peptides can be determined by comparing sequence information using a GAP computer program such as Needleman et al. (1970), J Mol Biol. 48: 443, for example, as described in Smith and Waterman, Adv. Appl. Math (1981) 2:482. In summary, the GAP program is defined as the total number of symbols in the shorter of two sequences divided by a number of similarly aligned symbols (that is, amino acids). Default parameters for the GAP program may include: (1) an unary comparison matrix (containing values of 1 for identity and 0 for non-identity) and a weighted comparison matrix (or EDNAFULL (EMBOSS version of NCBI NUC4.4) substitution matrix) of Gribskov et al (1986) Nucl. Acids Res. 14: 6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10, and a gap extension penalty of 0.5); and (3) no penalty for end gaps. Thus, as used herein, the term "homology" or "identity" refers to relevance between sequences.

In one embodiment, the peptide may be used in the form of a peptide alone, or in the form of a polypeptide in which the peptide is repeated two or more times and linked.

Therefore, the pharmaceutical composition of claim 1, comprising a polypeptide in which the peptide is linked repeatedly.

In one embodiment, a peptide comprising the amino acid sequence of general formula 1 applied to the present invention can be prepared by a combination of various methods for preparing various peptides.

According to the length of the peptide of the present invention, it can be synthesized by a method well known in the art, for example, an automatic peptide synthesizer, or it can be produced by genetic engineering technology. Specifically, the peptides of the present invention may be prepared by a standard synthetic method, a recombinant expression system, or any other method in the art. Accordingly, the peptides according to the present invention can be synthesized by a number of methods including, but not limited to, for example, methods including:
(a) A method of synthesizing peptides stepwise or by fragment assembly by means of solid or liquid phase methods, and isolating and purifying the final peptide product; or
(b) A method of expressing a nucleic acid construct encoding the peptide in a host cell and recovering an expression product from a host cell culture; or
(c) A method of performing cell-free in vitro expression of a nucleic acid construct encoding the peptide and recovering the expression product; or

A method of obtaining a fragment of a peptide by any combination of (a), (b) and (c), and then ligating the fragments to obtain a peptide, and recovering the peptide.

In addition, the preparation of the peptide may include modification with L- or D-form amino acids, and/or non-naturally occurring amino acids; and/or by modifying naturally occurring sequences, e.g., modification of side chain functional groups, intramolecular covalent bonds, such as inter-side chain ring formation, methylation, acylation, ubiquitination, phosphorylation, aminohexanation, biotinylation, etc. Also, the above modifications include all substitutions with non-naturally occurring compounds.

Substituted or added amino acids used in the above modifications may use atypical or non-naturally occurring amino acids as well as the 20 amino acids commonly found in human proteins. Commercial sources of atypical amino acids may include but are not limited to, Sigma-Aldrich, ChemPep, and Genzyme pharmaceuticals. Peptides including these amino acids and canonical peptide sequences, may be synthesized and purchased from commercial peptide synthesis companies, for example, American Peptide company or Bachem in the United States, or Anygen in Korea, but is not limited thereto.

Amino acid derivatives can also be obtained in the same manner, and 4-imidazoacetic acid can be used to name just a few examples.

In addition, a peptide according to the present invention may have an unmodified N-terminus and/or C-terminus, but in order to protect from proteolytic enzymes in vivo and increase stability, a modified form in which the N-terminus and/or C-terminus thereof are chemically modified or protected with an organic group, or amino acid is added to the peptide terminus, is also included in the scope of the peptide according to the present invention. When the C-terminus is not modified, a terminus of the peptide according to the present invention has a free carboxyl group but is not particularly limited thereto.

In particular, in the case of a chemically synthesized peptide, since the N- and C-terminals are charged, an N-terminus may be acetylated and/or a C-terminal may be amidated to remove these charges, but it is not particularly limited thereto.

The peptide may include a peptide having increased structural stability to heat, pH, etc., due to mutation or modification in an amino acid sequence, an increased prevention or treatment effect for periodontal disease or the dislocated traumatic tooth, or an increased ability to regenerate a periodontal ligament tissue.

The peptide includes both the peptide itself, a salt thereof (for example, a pharmaceutically acceptable salt of the peptide), or a solvate thereof.

A type of the salt is not particularly limited. However, it is preferably in a form that is safe and effective for an individual, such as a mammal but is not particularly limited thereto.

In addition, the peptide may be in any pharmaceutically acceptable form.

"Pharmaceutically acceptable" means a sufficient amount to exhibit a therapeutic effect and does not cause side effects, and it can be easily determined by a person skilled in the art according to factors well known in the medical field, including a type of disease, the patient's age, weight, health, or sex, patient's sensitivity to the drug, a route of administration, an administration method, a number of administration, a duration of treatment, and a drug used in combination or concurrently.

In one embodiment, the peptide may be in the form of a pharmaceutically acceptable salt thereof. The salts include conventional acid addition salts used in the pharmaceutical field, for example, in the field of periodontal disease, for example, salts derived from inorganic acids such as hydrochloric acid, hydrobromic acid, sulfuric acid, sulfamic acid, phosphoric acid, or nitric acid, and salts derived from organic acids such as acetic acid, propionic acid, succinic acid, glycolic acid, stearic acid, citric acid, maleic acid, malonic acid, methanesulfonic acid, tartaric acid, malic acid, phenylacetic acid, glutamic acid, benzoic acid, salicylic acid, 2-acetoxybenzoic acid, fumaric acid, toluenesulfonic acid, oxalic acid, or trifluoroacetic acid. In addition, the salt may be a base addition salt such as ammonium, dimethylamine, monomethylamine, monoethylamine, or diethylamine. The salts also include conventional metal salt forms, for example, salts derived from metals such as lithium, sodium, potassium, magnesium, or calcium. The acid addition salt, base addition salt or metal salt may be prepared according to a conventional method. Pharmaceutically acceptable salts and general methodologies for their preparation are well known in the art. See, for example, P. Stahl, et al. Handbook of Pharmaceutical Salts: Properties, Selection, and Use, 2nd Revised Edition (Wiley-VCH, 2011)]; [S.M. Berge, et al., "Pharmaceutical Salts," Journal of Pharmaceutical Sciences, Vol. 66, No. 1, January 1977.

For the condensation of a protected amino acid or peptide, various activating reagents useful for a peptide synthesis, particularly preferably a triphosphonium salt, a tetramethyluronium salt, carbodiimide, and the like can be used. Examples of triphosphonium salts include benzotriazol-1-yloxytris(pyrrolagino)phosphonium hexafluorophosphate (PyBOP), bromotris(pyrrolazino)phosphonium hexafluorophosphate (PyBroP), and 7 -azabenzotriazol-1-yloxytris(pyrrolazino)phosphonium hexafluorophosphate (PyAOP), examples of tetramethyluronium salts are 2-(1H-benzotriazol-1-yl)- 1,1,3,3-tetramethyluronium hexafluorophosphate (HBTU), 2-(7-azabenzotriazol-1-yl)-1,1,3,3-tetramethyluronium hexafluorophosphate (HATU), 2-(1H-benzotriazol-1-yl)-1,1,3,3-tetramethyluronium tetrafluoroborate (TBTU), 2-(5-norbornan-2,3 -dicarboxyimide)-1,1,3,3-tetramethyluronium tetrafluoroborate (TNTU), and O-(N-succimidyl)-1,1,3,3-tetramethyluronium tetrafluoroborate (TSTU), and examples of carbodiimides include N,N'-dicyclohexylcarbodiimide (DCC), N,N'-diisopropylcarbodiimide (DIPCDI), and N-ethyl-N'-(3-dimethylaminopropyl)carbodiimide hydrochloride (EDCI·HCl). For condensation using them, racemization inhibitors [for example, N-hydroxy-5-norbornen-2,3-dicarboxylic acid imide (HONB), 1-hydroxybenzotriazole (HOBt), 1-hydroxy-7-azabenzotriazole (HOAt), 3,4-dihydro-3-hydroxy-4-oxo-1,2,3-benzotriazine (HOOBt), and ethyl 2-cyano-2- (hydroxyl amino)acetate (Oxyma), etc.] may be added. A solvent used for condensation may be appropriately selected from those known to be useful for a peptide condensation reaction. For example, acid amides such as anhydrous or water-containing N,N-dimethylformamide, N,N-dimethylacetamide, and N-methylpyrrolidone, etc.; halogenated hydrocarbons such as methylene chloride, chloroform, etc.; alcohols such as trifluoroethanol, phenol, etc., sulfoxides such as dimethylsulfoxide, etc.; tertiary amines such as pyridine, etc.; ethers such as dioxane, tetrahydrofuran, etc.; nitriles such as acetonitrile, propionitrile, etc.; esters such as methyl acetate, ethyl acetate, etc.; and suitable mixtures thereof can be used. A reaction temperature is appropriately selected from the known ranges usable for a peptide binding reaction and is usually selected from a range of about -20 °C to 90 °C. Activated amino acid derivatives are usually used in 1.5 to 6-fold excess. In a solid phase synthesis, when the test using the ninhydrin reaction indicates that the condensation is insufficient, sufficient condensation can be performed by repeating the condensation reaction without removing a protecting group. When the condensation is still insufficient after repeating the reaction, an unreacted amino acid may be acetylated with an acid anhydride, acetyl imidazole, or the like, so that influence on a subsequent reaction can be avoided.

Examples of protecting groups for the amino group of a starting amino acid include benzyloxycarbonyl (Z), tert-butoxycarbonyl (Boc), tert-pentyloxycarbonyl, isobornyloxycarbonyl, 4-methoxybenzyloxycarbonyl, 2-chlorobenzyloxycarbonyl (Cl-Z), 2-bromobenzyloxycarbonyl (Br-Z), adamantyloxycarbonyl, trifluoroacetyl, phthaloyl, formyl, 2-nitrophenylsulfenyl, diphenylphosphinothioyl, 9-fluorenylmethyloxycarbonyl (Fmoc), trityl, and the like.

Examples of a carboxyl-protecting group for the starting amino acid include, in addition to the above-mentioned C₁₋₆ alkyl group, C₃₋₁₀ cycloalkyl group, and C₇₋₁₄ aralkyl group, aryl, 2-adamantyl, 4-nitrobenzyl, 4-methoxybenzyl, 4-chlorobenzyl, phenacyl, benzyloxycarbonylhydrazide, tert-butoxycarbonylhydrazide, tritylhydrazide, and the like.

A hydroxyl group of serine or threonine may be protected, for example, by esterification or etherification. Examples of groups suitable for esterification include lower (C2-4) alkanoyl groups such as acetyl groups; aroyl groups such as benzoyl groups; and groups derived from organic acids, and the like. Also, examples of suitable groups for etherification include benzyl, tetrahydropyranyl, tert-butyl (Bu^{t}), trityl (Trt), and the like.

Examples of protecting groups for a phenolic hydroxyl group of tyrosine include Bzl, 2,6-dichlorobenzyl, 2-nitrobenzyl, Br-Z, tert-butyl, and the like.

Examples of protecting groups for imidazole of histidine include p-toluenesulfonyl (Tos), 4-methoxy-2,3,6-trimethylbenzenesulfonyl (Mtr), dinitrophenyl (DNP), benzyloxymethyl (Bom), tert-butoxymethyl (Bum), Boc, Trt, Fmoc, and the like.

Examples of protecting groups for the guanidino group of arginine include Tos, Z, 4-methoxy-2,3,6-trimethylbenzenesulfonyl (Mtr), p-methoxybenzenesulfonyl (MBS), 2,2,5,7,8-pentamethylchroman-6-sulfonyl (Pmc), mesitylene-2-sulfonyl (Mts), 2,2,4,6,7-pentamethyldihydrobenzofuran-5-sulfonyl (Pbf), Boc, Z, NO₂, and the like.

Examples of protecting groups for a side chain amino group of lysine include Z, Cl-Z, trifluoroacetyl, Boc, Fmoc, Trt, Mtr, 4,4-dimethyl-2,6-dioxocyclohexylidenyl (Dde), and the like.

Examples of protecting groups for indolyl of tryptophan include formyl (For), Z, Boc, Mts, Mtr, and the like.

Examples of protecting groups for asparagine and glutamine include Trt, xantyl (Xan), 4,4'-dimethoxybenzhydryl (Mbh), 2,4,6-trimethoxybenzyl (Tmob), and the like.

Examples of activated carboxyl groups in a starting material include corresponding acid anhydride, azide, active esters [esters with alcohols (such as pentachlorophenol, 2,4,5-trichlorophenol, 2,4-dinitrophenol, cyanomethyl alcohol, paranitrophenol, HONB, N-hydroxysuccinimide, 1-hydroxybenzotriazole (HOBt), 1-hydroxy-7-azabenzotriazole (HOAt))], and the like. Examples of activated amino groups in a starting material include corresponding phosphorus amides.

Examples of methods for removing (eliminating) protecting groups include catalytic reduction in a hydrogen stream in the presence of a catalyst such as Pd-black or Pd-carbon; acid treatment with anhydrous hydrogen fluoride, methanesulfonic acid, trifluoromethanesulfonic acid, trifluoroacetic acid (TFA), trimethylsilyl bromide (TMSBr), trimethylsilyl trifluoromethanesulfonate, tetrafluoroboric acid, tris(trifluoro)boric acid, boron tribromide, or a mixed solution thereof; base treatment with diisopropylethylamine, triethylamine, piperidine, piperazine or the like; and reduction with sodium in liquid ammonia and the like. The removal reaction by acid treatment described above is generally performed at a temperature of -20 °C to 40 °C; and the reaction is efficiently performed by adding an acid treatment include anisole, phenol, thioanisole, methacresol and paracresol; and a cation scavenger such as dimethyl sulfide, 1,4-butanedithiol, 1,2-ethanedithiol, triisopropylsilane, and the like. Further, a 2,4-dinitrophenyl group used as a protecting group of imidazole of histidine is removed by a thiophenol treatment; a formyl group used as a protecting group of indole of tryptophan is removed by deprotection not only by acid treatment in the presence of 1,2-ethanedithiol, 1,4-butanedithiol, or the like, but also by alkali treatment with diluted sodium hydroxide, diluted ammonia, or the like.

Protection of a functional group that needs not to be involved in the reaction of a starting material with a protecting group, removal of a protecting group, activation of a functional group involved in the reaction, and the like may be appropriately selected from known protecting groups and known means.

In a method for preparing an amide of a peptide, it is formed by a solid phase synthesis using a resin for amide synthesis, or the α-carboxyl group of a carboxy-terminal amino acid is amidated, and a peptide chain is extended to the desired chain length toward an amino group, thereafter, a peptide in which a protecting group for an N-terminal α-amino group of only the peptide chain has been removed and the peptide with only a protecting group for the C-terminal carboxyl group removed from the peptide chain are prepared, and these two peptides are condensed in a solvent mixed as described above. For the details of a condensation reaction, the same applies as above. After a protected peptide obtained by condensation is purified, all protecting groups can be removed by the method described above to obtain the desired crude peptide. A desired amide of the peptide can be prepared by purifying this crude peptide using various publicly known means of purification and freeze-drying of the main fraction.

In one embodiment, the peptide may be in the form of a solvate thereof. "Solvate" means that the peptide or a salt thereof forms a complex with a solvent molecule.

"Periodontal disease" refers to an inflammatory disease that occurs in the tissues, such as gingiva, periodontal ligament, and alveolar bone around a tooth that holds a tooth. Periodontal disease refers to a disease in which bacteria infect the gap between the gingiva and teeth and damage the periodontal ligament and adjacent tissues, and according to the severity of the disease, it is divided into gingivitis and periodontitis. As inflammation progresses, it is known that, as inflammation progresses and more tissue is damaged, a periodontal pocket is formed, and as periodontitis is severe and the periodontal pocket deepens, so that the periodontal ligament becomes inflamed and eventually bone loss is induced. Since a fundamental treatment of such periodontal disease is to restore the damaged periodontal ligament a connective tissue, cementum, and alveolar bone, for this purpose, not only the periodontal ligament supporting the alveolar bone needs to be regenerated, but also alveolar bone and cementum to which the periodontal ligament can be attached are necessary.

In the pharmaceutical composition, periodontal disease may be a periodontium inflammatory disease.

In the pharmaceutical composition, periodontal disease may be gingivitis or periodontitis.

In one embodiment, the pharmaceutical composition may further include a drug for treating the periodontal disease.

A drug for treating the periodontal disease may be separated from the peptide, or may be in a form in which a complex is formed with the peptide by binding to the N-terminus or C-terminus of the peptide.

"Dislocated trauma" includes trauma in which a traumatic force is applied to a tooth or periodontium, and the tooth and periodontium are destroyed according to the difference in direction and size, and the periodontal ligament is damaged, including those subdivided into concussive subdislocation, orbital dislocation, lateral dislocation, complete dislocation, and intrusion. Therefore, a fundamental treatment of the dislocated trauma also requires regeneration of damaged periodontal ligament. In particular, the success of replanting a completely dislocated tooth depends on the regeneration of the periodontal ligament attached to the completely dislocated tooth or the periodontal ligament remaining in the alveolar fossa from which the tooth is missing. When periodontal ligament regeneration does not occur, the cementum and dentin of the tooth root are absorbed, and ankylosis occurs in which this region is replaced by alveolar bone.

"Tooth replantation" is a method of re-transplanting an extraction socket after intentionally extracting a tooth or performing or prior to performing an appropriate root canal treatment. Intentional replantation can be performed in situations where root canal treatment has failed, anatomical limitations, accessibility difficulties, dislocated teeth due to an accident, or intentionally rapid orthodontic eruption is required.

"Prevention" refers to any action that suppresses or delays the onset of the periodontal disease or the dislocated traumatic tooth by administration of the composition.

"Treatment" refers to any action in which symptoms of the periodontal disease or the dislocated traumatic tooth are improved or beneficial by administration of the composition.

The pharmaceutical composition may be prepared in the form of a pharmaceutical composition for treating the periodontal disease, further including an appropriate carrier (natural or non-natural carrier), excipient or diluent commonly used in the preparation of pharmaceutical compositions to the peptide. The pharmaceutical composition may be formulated and used in the form of a sterile injectable solution that can be administered to the area induced by the periodontal disease according to a conventional method, respectively.

In the present invention, carriers, excipients and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, collagen, etc. In the case of formulation, it can be prepared using diluents or excipients such as fillers, extenders, binders, wetting agents, disintegrating agents and surfactants usually used. In particular, sterile aqueous solutions, non-aqueous solutions, suspensions, emulsions, lyophilized preparations, suppositories, and ointments (e.g., dimensional liner, etc.), etc. may be included. As the nonaqueous solvent and suspending agent, propylene glycol, polyethylene glycol, vegetable oils such as olive oil, injectable esters such as ethyl oleate, etc., can be used. As the suppository base, Witepsol, Macrogol, Tween 61, cacao butter, laurin butter, glycerogeratin, etc. can be used.

Content of a peptide or a pharmaceutically acceptable salt thereof contained in the pharmaceutical composition of the present invention is not particularly limited but may be contained in an amount of 0.0001 to 50 wt %, or 0.01 to 20 wt %, based on the total weight of the final composition.

The pharmaceutical composition can be administered in a pharmaceutically effective amount, and the term "pharmaceutically effective amount" means an amount sufficient to treat or prevent a disease at a reasonable benefit/risk ratio applicable to medical treatment or prevention and an effective dose level can be determined according to severity of a disease, an activity of a drug, age, body weight, health, sex of a patient, sensitivity to patient's drugs, time of administration, route of administration and duration of treatment of a rate of excretion of the inventive composition employed, factors including the drugs used in combination or coincidental with the inventive compositions employed, and other factors well known in the medical arts. The pharmaceutical composition of the present invention may be administered alone or in combination with known pharmaceutical compositions for treating periodontal disease. Considering all of the above factors, it is important to administer a minimum amount of the maximum effect without side effects.

The dosage of the pharmaceutical composition can be determined by those skilled in the art in consideration of the purpose of use, the severity of the disease, the age, body weight, sex, history of the patient, or the kind of substance used as an active component. For example, the pharmaceutical composition of the present invention can be administered at about 0.1 ng to about 100 mg/kg, preferably 1 ng to 10 mg/kg, per adult, and the dosage frequency of the composition is not particularly limited but may be administered once a day or several divided doses. The above dosages do not limit the scope of the invention in any respect.

Another aspect of the present invention for solving the above technical problem provides a composition for regenerating periodontium, including a peptide comprising an amino acid sequence of the following general formula 1:

K-Y-K-Q-X5-X6-X7-X8-Y-K (General formula 1)

in the general formula 1,
X5 to X7 are each independently arginine (R) or lysine (K);
X8 is asparagine (N) or serine (S).

In one embodiment, the peptide includes the amino acid sequence of any one of SEQ ID NOs: 1 to 16.

In another embodiment, the peptide consists essentially of an amino acid sequence of any one of SEQ ID NOs: 1 to 16, or the peptide consists of an amino acid sequence of any one of SEQ ID NOs: 1 to 16.

The description of the peptide is as described above.

"Periodontium" is a complex organ composed of epithelial tissue, a connective tissue (soft tissue), and calcified connective tissue, and is structurally composed of the gingiva, periodontal ligament (PDL), cementum, and alveolar bone.

"Periodontal ligament (PDL)", also called periodontium, refers to a connective tissue fibrous membrane that connects the cementum of tooth root and a wall of alveolar bone in mammals. The periodontal ligament is composed of a main fiber, a collagen fiber extending parallel or obliquely to the long axis of the tooth, and a bundle of Sharpey fibers, both ends of which are continuously buried in hard tissue, and the tooth is elastically fixed to alveolar bone through the periodontal ligament. The periodontal ligament not only buffers the pressure generated when chewing food but also has abundant blood vessels and nerves and is known to be involved in nutrition and sensation. The periodontal ligament contains a variety of cells such as fibroblast, undifferentiated mesenchymal cells, and epithelial cells, among them, periodontal ligament fibroblast can be differentiated into osteoblast or cementoblast by appropriate stimulation, but a mechanism of differentiation of periodontal ligament fibroblast into cementoblast and differentiation-promoting proteins are not known. Bone sialoprotein (BSP), osteocalcin (OC), CAP (cementum attachment protein), etc., are known as markers of cementoblast differentiation, in particular, CAP is known to play an important role in the attachment of periodontal ligament fibers to the cementum.

"Cementum" is a calcified tissue covering the root of teeth in mammals. The cementum anchors a tooth to the alveolar bone by immobilizing the periodontal ligament. Therefore, when bacteria are infected with gingiva, the cementum surrounding the tooth degenerates, and the periodontal ligament fiber bundle that connects the tooth and alveolar bone cannot adhere to the degenerated cementum, causing the tooth to shake. The formation of new cementum is necessary for treating such degenerated cementum.

"Alveolar bone" is a part of an upper jaw and a lower jaw that surrounds a tooth root and supports a tooth, and develops according to the formation or eruption of teeth, and is gradually absorbed when teeth are lost. By fixing the periodontal ligament together with root cementum, the alveolar bone plays an important role in occlusal pressure, such as mastication, as well as dispersion and absorption of pressure on a tooth during pronunciation and swallowing.

"Periodontal ligament fibroblast" together with gingival fibroblast is the main cellular constituent of connective tissue (soft tissue) of the periodontium. The periodontal ligament fibroblast not only forms the periodontal ligament as a unique function thereof, but is also involved in the repair and regeneration of an adjacent alveolar bone and cementum in vivo, but there is no known material that can promote this, and it is distinct from gingival fibroblast, which are involved in maintaining a gingival connective tissue. Periodontal ligament fibroblast can be differentiated into osteoblast or cementoblast by appropriate stimulation, but the differentiation mechanism of the periodontal ligament fibroblast into cementoblast is not clearly known. Bone sialoprotein (BSP), osteocalcin (OC), or the like are known as osteoblast and cementoblast differentiation markers, and CAP (cementum attachment protein) is known as a gene that plays an important role in the attachment of periodontal ligament fibers to cementum. Also, a periostin gene is known as a periodontal ligament marker. According to several recent studies, it has been reported that periostin plays an important role in the regeneration of periodontal ligament and alveolar bone after periodontal surgery by promoting collagen fiber production and migration of fibroblast and osteoblast as an important regulator of periodontium formation.

"Regeneration" may refer to any action in which lost or damaged cells or tissues are repaired or replenished. The regeneration may be due to cell differentiation.

It was confirmed that the peptide has an effect of promoting the differentiation of periodontal ligament fibroblast into cementoblast.

A composition for regenerating periodontium may promote the regeneration of any one or more of the gingiva, periodontal ligament, cementum, and alveolar bone.

A composition for regenerating periodontium may be a pharmaceutical composition.

The pharmaceutical composition is as described above.

A composition for regenerating periodontium may be a quasi-drug composition. The quasi-drug composition may be quasi-drug composition for preventing or improving the periodontal disease or the dislocated traumatic tooth.

The term "improvement" refers to any act of reducing at least the extent of a parameter, e.g., a symptom, associated with a condition being treated.

The improvement can be interpreted as meaning an action that promotes the regeneration of periodontium by administering a pharmaceutical composition comprising a peptide as an active component to an individual requiring treatment of the periodontal disease, thereby improving or benefiting the symptoms of the periodontal disease or the dislocated traumatic tooth.

The term "quasi-drug" refers to articles that are milder in action than pharmaceuticals, among articles used for the purpose of diagnosing, treating, ameliorating, alleviating, treating or preventing a disease in a human or animal, for example, according to the pharmacist method, the quasi-drug is a substance other than an article used for pharmaceutical and includes a fiber/rubber product used for treating or preventing diseases of humans/animals, a substance similar to a substance that has little or no direct action on the human body and is not a mechanism or a machine, and a bactericidal/pesticidal agent for preventing infectious diseases.

The type or formulation of the quasi-drug composition comprising the peptide is not particularly limited, but as an example, it may be an oral disinfectant, an oral cleaning product, a toothpaste, a dental floss, an oral ointment, and the like.

The composition for regenerating periodontium may be a healthy functional food composition. The health functional food composition may be a health functional food composition for preventing or improving periodontal disease or a dislocated traumatic tooth.

"Food" of the present invention includes dairy products, including meat, sausage, bread, chocolate, candies, snacks, confectionary, pizza, ramie, other noodles, gums, ice cream, various soups, beverages, teas, drinks, alcohol drinks, vitamin complexes, health functional foods, and health foods, etc., and includes all foods in a conventional sense.

The above health functional food is the same term as the food for special health use (FoSHU), and means a food product having a high medical and medical effect processed so that bioregulatory functions are efficiently exhibited in addition to nutrient supply. The term "function(ality)" as used herein, means to control nutrients for the structure and function of the human body or to obtain a useful effect for health uses such as physiological action.

The food of the present invention can be produced by a method commonly used in the art, and in such production, raw materials and components commonly added in the industry can be added. The formulation of the food can also be prepared without limitation, provided that it is a formulation recognized as food. The composition for food of the present invention can be prepared in various forms of dosage form, has the advantage of using food as a raw material, unlike general drugs and having no side effects, etc. that can occur during long-term dosing of drugs, and is excellent in carrying property, and the food of this invention can also be taken as an adjuvant for enhancing the effect of preventing or improving the periodontal disease or the dislocated traumatic tooth.

The term "health functional food" refers to a food product having active health maintenance and an enhancing effect as compared with the general formula product, and a health supplement food refers to a food product for a health supplement purpose. In some cases, the terms health functional food, health food, and health supplement food may be used interchangeably.

Specifically, the above health functional food means a food product prepared by adding a peptide to a food material such as a beverage, a tea, a flavoring agent, a gum, and a confectionery, or by encapsulating, pulverizing, suspending or the like, which brings about a certain effect on health when ingested, but it has an advantage that it does not have any adverse effect that can occur when a food product is used as a raw material for a long-term administration of a drug, unlike general drugs.

Since the food composition of the present invention can be consumed on a daily basis, a high effect can be expected for the prevention or improvement of periodontal disease or the dislocated traumatic tooth, and thus it can be very usefully used.

The food composition may further comprise a physiologically acceptable carrier, and the kind of the carrier is not particularly limited, and any carrier commonly used in the art may be used.

In addition, the food composition may include additional components that are commonly used in food compositions to enhance odor, taste, vision, etc. For example, vitamins A, C, D, E, B1, B2, B6, B12, niacin, biotin, folate, pantothenic acid, and the like may be included. It may also include minerals such as zinc (Zn), iron (Fe), calcium (Ca), chromium (Cr), magnesium (Mg), manganese (Mn), and copper (Cu). It may also contain amino acids such as lysine, tryptophan, cysteine, valine, or the like.

In addition, the food composition may contain food additives such as antiseptics (potassium sorbate, sodium benzoate, salicylic acid, sodium dehydrocholate, etc.), bactericides (bleaching powder, high bleaching powder, sodium hypochlorite, etc.), antioxidants (butylhydroxyanisole (BHA), butylhydroxytolene (BHT), etc.) coloring agents (tar coloring agent, etc.), color-forming agents (sodium nitrite, sodium subacetate, etc.). Bleaching agent (sodium sulfite), seasonings (sodium MSG glutamate, etc), sweeteners (dulcine, cyclamate, saccharin, sodium, etc) perfumes (vanillin, lactones, etc); expanding agents (alum, potassium D-tartrate, etc); reinforcing agents, emulsifiers, thickeners (dements), coating agents, screening agents, foam inhibitors, solvents, and conditioners. The additives are selected according to the kind of food and can be used in an appropriate amount.

The peptide can be added as it is or used in combination with other food or food components and can be suitably used according to a conventional method. The mixed amount of the active component can be suitably determined according to the purpose of its use (prevention, health or therapeutic treatment). Generally, in the manufacture of a food or beverage, the food composition of the present invention may be added in an amount of 50 parts by weight or less, 20 parts by weight or less, 10 parts by weight or less, 5 parts by weight or less, 1 parts by weight or less, or 0.1 parts by weight or less. However, when ingested for a long period of time for the purpose of health and hygiene, the content may be within the above range, and since there is no problem in terms of safety, the active component may also be used in an amount above the above range.

One example of the food composition may be used as a health beverage composition, in which case it may contain as additional components various flavoring agents, natural carbohydrates, etc., such as conventional beverages. The above-mentioned natural carbohydrates may be monosaccharides such as glucose, and fructose; disaccharides such as maltose, and sucrose; polysaccharides such as dextrin, and cyclodextrin; sugar alcohols such as xylitol, sorbitol, erythritol, etc. As the sweetening agent, natural sweetening agents such as thaumatin, and stevia extract; synthetic sweeteners such as saccharin, aspartame, etc. can be used. The proportion of natural carbohydrates can generally be about 0.01 to 0.04 g, specifically about 0.02 to 0.03 g, per 100 ml of the health drink composition of the present invention.

In addition to the above, the health drink composition may contain various nutrients, vitamins, electrolytes, flavoring agents, coloring agents, pectic acid, salts of pecting acid, alginic acid, salt of alginic acids, organic acids, protective colloid thickeners, pH regulators, stabilizers, antiseptics, glycerin, alcohols, carbonating agents, etc. They may also contain flesh for the production of natural fruit juices, fruit juice beverages, or vegetable beverages. These components may be used independently or in combination. The proportion of such additives is not critical but is generally selected in the range of 0.01 to 0.1 parts by weight per 100 parts by weight of the health and beverage composition of the invention.

The food composition may comprise various weight %s as long as the effect of preventing or ameliorating the periodontal disease or the dislocated traumatic tooth is exhibited, but specifically, it may comprise 0.00001 to 100 weight % or 0.01 to 80 weight % of the peptide of the present invention, relative to the total weight of the food composition, but is not limited thereto.

Another aspect of the present invention for solving the above technical problem provides a composition for promoting the expression of any one or more genes of bone sialoprotein (BSP), dentin matrix protein 1 (DMP1), Cementum attachment protein (CAP), collagen type III (COL3) and Periostin, including a peptide comprising an amino acid sequence of the following general formula 1:

K-Y-K-Q-X5-X6-X7-X8-Y-K (General formula 1)

in the general formula 1,
X5 to X7 are each independently arginine (R) or lysine (K);
X8 is asparagine (N) or serine (S).

In one embodiment, the peptide includes an amino acid sequence of any one of SEQ ID NOs: 1 to 16.

In another embodiment, the peptide consists essentially of an amino acid sequence of any one of SEQ ID NOs: 1 to 16, or the peptide consists of an amino acid sequence of any one of SEQ ID NOs: 1 to 16.

The description of the peptide is as described above.

It was confirmed that the peptide increased the expression of BSP, DMP1, and CAP, which are marker genes for osteoblast and cementoblast differentiation, and increased the expression of periostin and COL3, which are marker genes for periodontal ligament differentiation in human periodontal ligament cells. In addition, it was confirmed that the peptide increases the expression of BSP and DMP1, which are marker genes for osteoblast and cementoblast differentiation, in human mesenchymal stem cells.

Another aspect for solving the technical problem of the present invention is to provide a method for treating the periodontal disease or the dislocated traumatic tooth in an individual, the method includes administering an effective amount of a peptide or a pharmaceutical acceptable salt thereof to an individual in need of treatment of the periodontal disease or the dislocated traumatic tooth.

"Individual" means a subject in need of treatment for a disease, and more specifically, mammals such as human or non-human primates, mice, rats, dogs, cats, horses and cattle.

"Effective amount" refers to an amount or dose of the peptide or a pharmaceutically acceptable salt thereof that, when administered to a patient in single or multiple doses, provides the desired effect in the patient under diagnosis or treatment. An effective amount can be readily determined by the attending physician of ordinary skill in the art by using known techniques or by observing the results obtained under similar circumstances. When determining an effective amount for a patient, a number of factors are considered by the attending physician, including, but not limited to species, size, age, and general health of mammalian; specific disease or disorder involved; degree or severity of involvement of the disease or disorder; individual patient response; the particular compound being administered; mode of administration; bioavailability characteristics of an agent being administered; selected dosing regimen; use of concomitant medications; and other relevant circumstances.

By "administration" is meant introducing a substance into a patient by any appropriate method. A route of administration may be any general route that can reach the patient's in vivo target. The administration includes but is not limited to, for example, intraperitoneal administration, intravenous administration, intramuscular administration, subcutaneous administration, intradermal administration, oral administration, topical administration, intranasal administration, and intrarectal administration.

Treatment of periodontal disease or the dislocated traumatic tooth includes, but is not limited to, by regeneration of periodontium.

In the method, an effective amount of the peptide or a pharmaceutically acceptable salt thereof may be administered simultaneously, separately, or sequentially with an effective amount of one or more other active components. The one or more other active components include, but are not limited to, one or more other agents for treating the periodontal disease or the dislocated traumatic tooth.

Another aspect of the present invention for solving the above technical problem is to provide a use of a peptide or a pharmaceutically acceptable salt thereof in the manufacture of a medicament for preventing or treating periodontal disease or dislocated traumatic tooth.

All numerical values disclosed herein may include the meaning of "about." "About" is a range inclusive of ±0.5, ±0.4, ±0.3, ±0.2, ±0.1, and the like, and includes, but is not limited to, all values within a range equivalent to or similar to the value following the term about.

Meanwhile, each description and embodiment disclosed herein may also be applied to other descriptions and embodiments. That is, all combinations of the various elements disclosed herein are within the scope of the present invention. In addition, it cannot be said that the scope of the present invention is limited by the specific descriptions described below.

### [Effects of the Invention]

The pharmaceutical composition for preventing or treating a periodontal disease or a dislocated traumatic tooth according to the present invention, promotes the regeneration of periodontium, including periodontal ligament, alveolar bone, and cementum, and adhesion of periodontal ligament so that the composition can also be used to restore damaged connective tissue such as the periodontal disease and a dislocated traumatic tooth. Accordingly, the peptide can be used in various ways as pharmaceutical compositions, quasi-drugs compositions, health functional food compositions, and the like.

The effects of the present invention are not limited to those mentioned above, and other effects not mentioned may be clearly understood by those skilled in the art from the following description.

### [Description of Drawings]

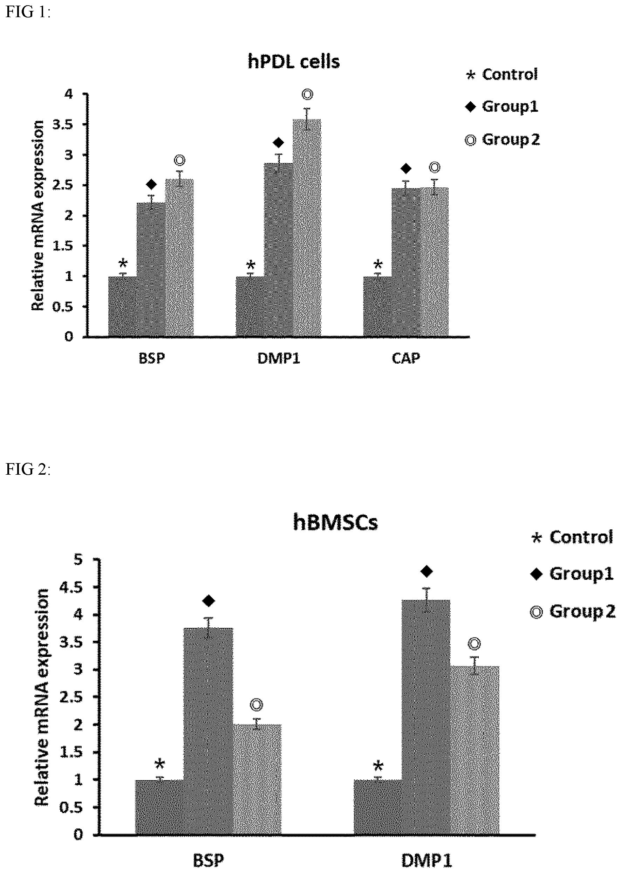

FIG. 1 is a graph showing an effect of peptides of group 1 and group 2 prepared in Example 1 on an expression of bone sialoprotein (BSP), dentin matrix protein 1 (DMP1), and CAP(Cementum attachment protein), which are marker genes for osteoblast and cementoblast differentiation in a human periodontal ligament cell (hPDL).
FIG. 2 is a graph showing an effect of peptides of group 1 and group 2 prepared in Example 1 on an expression of bone sialoprotein (BSP), and dentin matrix protein 1 (DMP1), which are marker genes for osteoblast differentiation in human mesenchymal stem cells(hBMSCs).

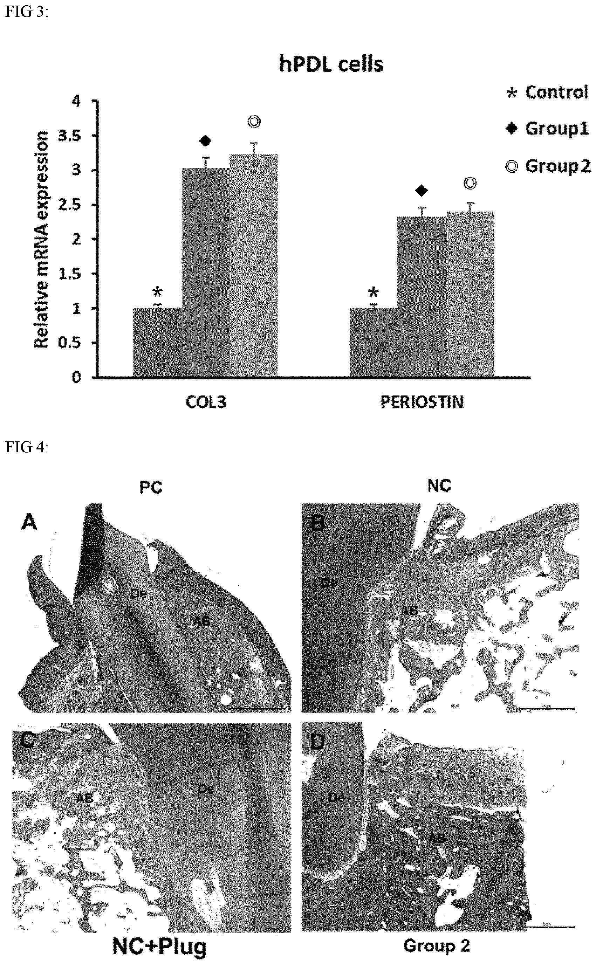

FIG. 3 is a result showing the effect of peptides of group 1 and group 2 prepared in Example 1 on expressions of type 3 collagen fiber gene (COL3) and periostin gene, which are periodontal ligament marker genes, in human periodontal ligament cells.
FIG. 4 is a photograph of tissue stained with hematoxylin/eosin after 3 months of treatment with the peptide (SEQ ID NO: 16) of Example 1 at the periodontium damaged region and shows the results of confirming an effect of a peptide on alveolar bone regeneration. A represents a positive control (PC); B represents a negative control (NC) without any treatment after periodontium damage; C represents a negative control (NC+Plug) transplanted with collagen sponge only after periodontium damage; D represents an experimental group treated with the peptide (SEQ ID NO: 16) of group 2; AB means an alveolar bone; De means dentin.

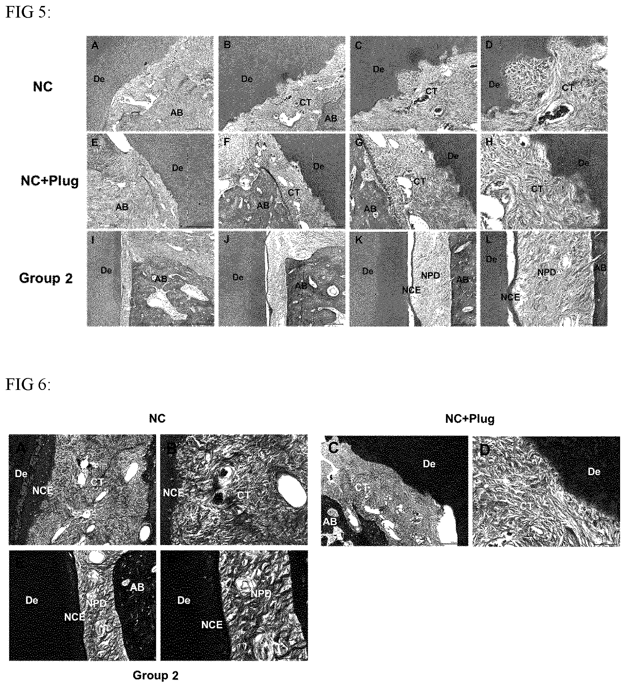

FIG. 5 the result of confirming an effect of the peptide on periodontal ligament tissue regeneration by staining with hematoxylin/eosin after 3 months of treatment with the peptide (SEQ ID NO: 16) of Example 1 at periodontium damage region. A-D represents a negative control (NC) without any treatment; E-H represents a negative control (NC+Plug) transplanted with collagen sponge only; and I-L represents an experimental group treated with a peptide (SEQ ID NO: 16) of group 2. AB means alveolar bone; De means dentin; CE means cementum; CT means connective tissue; NPD refers to a newly formed periodontal ligament-like tissue; and NCE means a newly formed cementum-like tissue. Size bars, A, E, I, M 500 µm. B, F, J, N, 200 µm. C, G, K, O, 100 µm. D, H, L, P, 50 µm.
FIG. 6 is the result of confirming the effect of the peptide on periodontal ligament tissue regeneration by a collagen staining (Masson's trichrome stain) method after 3 months of treatment with the peptide (SEQ ID NO: 16) of Example 1 at periodontium damage region. A-B represents a negative control (NC) without any treatment; C-D represents a negative control (NC+Plug) transplanted with collagen sponge only; and E-F represents an experimental group treated with a peptide (SEQ ID NO: 16) of group 2. AB means alveolar bone; De means dentin; CE means cementum; CT means connective tissue; NPD refers to a newly formed periodontal ligament-like tissue; NCE means a newly formed cementum-like tissue. Size bars, A, C, E, G 100 µm. B, D, F, H, 50 µm.

### [Modes of the Invention]

Hereinafter, the present invention will be described in detail by way of Examples. However, these Examples are for illustrative purposes of the present invention, and the scope of the present invention is not limited to these Examples.

### Example 1: Synthesis of peptides

16 peptides composed of amino acid sequences shown in Table 1 were synthesized, respectively. Here, a peptide of which an 8th amino acid is an asparagine (N) was group 1, and a peptide of which an 8th amino acid is serine (S) was group 2.

**[Table 1]**

| Group | SEQ ID NO | Amino acid sequence (N-C) |
|---|---|---|
| Group 1 | 1 | KYKQRKKNYK |
| | 2 | KYKQRKRNYK |
| | 3 | KYKQRRKNYK |
| | 4 | KYKQRRRNYK |
| | 5 | KYKQKKKNYK |
| | 6 | KYKQKRKNYK |
| | 7 | KYKQKKRNYK |
| | 8 | KYKQKRRNYK |
| Group 2 | 9 | KYKQRKKSYK |
| | 10 | KYKQRKRSYK |
| | 11 | KYKQRRKSYK |
| | 12 | KYKQRRRSYK |
| | 13 | KYKQKKKSYK |
| | 14 | KYKQKRKSYK |
| | 15 | KYKQKKRSYK |
| | 16 | KYKQKRRSYK |

### Experimental material and Method 1. Cell culture

hBMSCs cells were cultured under the condition of humidified air at 37°C containing 5% CO₂ and used in an experiment. Human-derived mesenchymal stem cells (hBMSCs) were purchased from Lonza (LONZA, Switzerland) and used. hBMSCs were cultured in α-MEM (Invitrogen) medium supplemented with 10% heat-inactivated bovine serum.

### 2. Human periodontal ligament cell isolation and culture

Human periodontal ligament cells (hPDL cells) were isolated from periodontal ligament tissues from the wisdom teeth of 10 adults (18-22 years old) at Seoul National University Dental Hospital. Specifically, all experiments were performed with the consent of the patient after obtaining approval from the Hospital's Institutional Review Board, a periodontal ligament tissue attached to the root of a wisdom tooth was isolated and cut into double-sided pieces, placed in a 60 mm dish, and covered with a cover slip, and then cultured in Dulbecco's degenerated Eagle's Medium.

### 3. Real-time PCR analysis

Total RNA from human periodontal ligament cells was isolated using a TRIzol reagent. cDNA was synthesized using 2 µg of total RNA, 1 µl of reverse transcriptase, and 0.5 µg of oligo (dT). cDNA of synthesized human periodontal ligament cells was used for a real-time polymerase chain reaction using primers shown in Table 2 below. A real-time polymerase chain reaction was performed in ABI PRISM 7500 sequence detection system (Applied Biosystems) using SYBR GREEN PCR Master Mix (Takara, Japan). The real-time polymerase chain reaction was performed at 94 °C, 1 min; 95 °C, 15 sec - 60 °C, 34 sec was performed under the condition of repeating 40 cycles. Results were analyzed using a comparative cycle threshold (CT) method.

**[Table 2]**

| Gene | SEQ ID NO | Direction | Sequence (5'-3') |
|---|---|---|---|
| *hBSP* | 17 | Forward | GAATGGCCTGTGCTTTCTCAA |
| | 18 | Reverse | TCGGATGAGTCACTACTGCCC |
| *hDMP1* | 19 | Forward | ACAGGCAAATGAAGACCC |
| | 20 | Reverse | TTCACTGGCTTGTATGG |
| *hCAP* | 21 | Forward | GACGAGGACGGCACCAACGG |
| | 22 | Reverse | CGCGGTCATGGCGATGTCGT |
| *hPERIOSTIN* | 23 | Forward | GAGACAAAGTGGCTTCCG |
| | 24 | Reverse | CTGTCACCGTCACATCCT |
| *hCollagen Type III* | 25 | Forward | CGGATGCTTCCAGACATCTCTATC |
| | 26 | Reverse | ACAGGAAGCTGTTGAAGGAGGA |
| *hGAPDH* | 27 | Forward | CCATGGAGAAGGCTGGGG |
| | 28 | Reverse | CAAAGTTCTCATGGATGACC |

### 4. Evaluation of the effect of peptides on periodontium regeneration in periodontium damage model

Four beagle dogs (12-16 kg; 6-8 weeks old) were anesthetized by inhalation of Geloran, intravenously injected with Zoletil (5 mg/kg) and Xylazine (0.2-0.5 mg/kg), and then treated with Lidocaine (Lidocaine 2% with 1:80,000 epinephrine). The 4th premolar tooth and the 2nd molar tooth were extracted from the mandible of the beagle dog, and then the dog waited for healing for 3 months.

After 3 months of healing, three-wall periodontal defect was induced on distal and mesial surfaces of a 1 st molar tooth. Then, a periodontal damage of 4x4x4 mm was formed with the buccal and distal bones remaining around the tooth root, the root alveolar bone is securely removed, tooth root planing was performed using a curette, and then an incisor groove was formed using a 1/2 round bur.

Next, an experimental group was transplanted with a collagen sponge (a peptide solution obtained by dissolving a peptide in physiological saline at a concentration of 1 µg/µl was prepared, 50 µl of a peptide solution was added to the EP tube, and then was soaked for 5 minutes to prepare the collagen sponge) including a 50 µl of peptide (SEQ ID NO: 16 of group 2) prepared in Example 1 at a defect region, After the defect, only a negative control (NC) without any treatment and a collagen sponge (prepared by soaking a collagen sponge in 50 µl of physiological saline for 5 minutes) was placed on the inner bone wall of a bone defect and a transplanted negative control (NC+Plug) was sacrificed 3 months later, and histological evaluation was performed.

After 3 months, a beagle dog was sacrificed by administering an excessive amount (90-120 mg/kg) of pentobarbital. A tooth of the beagle dog was extracted, fixed with 10% formalin, and then calcium was removed by adding 5% formic acid, molded, and embedded in paraffin, to obtain a tissue section with a thickness of 5 µm.

The obtained tissue section was stained with hematoxylin and eosin or collagen stained (Masson's Trichrome Staining) to confirm periodontal ligament regeneration and then analyzed using by an optical microscope equipped with a digital camera (LEICA ICC50 camera, Germany).

### Experimental Results

### Experimental Example 1: Effect of peptides on the expression of marker genes for osteoblast and cementoblast differentiation in human periodontal ligament cells

Bone sialoprotein (BSP) and dentin matrix protein 1 (DMP1) genes are used as markers for the differentiation of osteoblast and cementoblast and are known as important genes for the calcification of a bone and cementum. In addition, a CAP (cementum attachment protein) gene is expressed in differentiated cementoblast and is known as a gene involved in the cementum attachment of a periodontal ligament fiber bundle.

FIG. 1 is a graph showing the effect of peptides of group 1 and group 2 prepared in Example 1 on an expression of marker genes for osteoblast and cementoblast differentiation BSP, DMP1, and CAP in a human periodontal ligament cell (hPDL).

As shown in FIG. 1, in an experimental group treated with the peptides of group 1 and group 2, it was confirmed that the expression of BSP, DMP1, and CAP genes were increased by about two times or more compared to the control.

Table 3 shows the results of real-time PCR confirming the effect of the peptide groups of group 1 and group 2 on PCR mRNA expression and shows a relative mRNA expression level compared to the control.

Table 4 shows the results of real-time PCR confirming the effect of the peptide groups of group 1 and group 2 on CAP mRNA expression and shows a relative mRNA expression level compared to the control. The results shown in Tables 3 and 4 are the average value and standard deviation (SD) obtained by repeating the experiment three times.

**[Table 3]**

| BSP gene expression | | | |
|---|---|---|---|
| | SEQ ID NO | Average | SD |
| Group 1 | 1 | 2.092 | 0.152 |
| | 2 | 2.361 | 0.098 |
| | 3 | 2.572 | 0.209 |
| | 4 | 2.702 | 0.301 |
| | 5 | 2.67 | 0.088 |
| | 6 | 2.705 | 0.137 |
| | 7 | 2.215 | 0.072 |
| | 8 | 2.021 | 0.301 |
| Group 2 | 9 | 2.211 | 0.413 |
| | 10 | 2.811 | 0.302 |
| | 11 | 2.362 | 0.182 |
| | 12 | 2.211 | 0.287 |
| | 13 | 2.525 | 0.25 |
| | 14 | 2.836 | 0.099 |
| | 15 | 2.620 | 0.401 |
| | 16 | 2.606 | 0.371 |

**[Table 4]**

| CAP gene expression | | | |
|---|---|---|---|
| | SEQ ID NO | Average | SD |
| Group 1 | 1 | 2.092 | 0.152 |
| | 2 | 2.361 | 0.098 |
| | 3 | 2.572 | 0.209 |
| | 4 | 2.702 | 0.301 |
| | 5 | 2.67 | 0.088 |
| | 6 | 2.705 | 0.137 |
| | 7 | 2.451 | 0.072 |
| | 8 | 2.021 | 0.301 |
| Group 2 | 9 | 2.211 | 0.413 |
| | 10 | 2.811 | 0.302 |
| | 11 | 2.362 | 0.182 |
| | 12 | 2.211 | 0.287 |
| | 13 | 2.525 | 0.25 |
| | 14 | 2.836 | 0.099 |
| | 15 | 2.620 | 0.401 |
| | 16 | 2.467 | 0.371 |

### Experimental Example 2: Effect of peptides on the expression of marker genes for osteoblast and cementoblast differentiation in human mesenchymal stem cells

FIG. 2 is a result showing the effect of the peptides of group 1 and group 2 prepared in Example 1 on the expression of marker genes for osteoblast and cementoblast differentiation in human mesenchymal stem cells.

As shown in FIG. 2, it was confirmed that when human mesenchymal stem cells were treated with peptide group 1 and group 2, an expression of BSP and DMP1, which are marker genes for osteoblast and cementoblast differentiation, was about 2 to 4 times higher than that of a control.

### Experimental Example 3: Effect of peptides on the expression of marker genes for a periodontal ligament in human periodontal ligament cells

Periostin is a protein first discovered in a periodontal ligament and periosteum of a bone, and periostin is expressed in the periodontal ligament and mesenchymal of the developing tooth and is known to be involved in cell adhesion. According to several recent studies, periostin is an important regulator of periodontium formation, promotes collagen fiber production and migration of fibroblast and osteoblast, and it is known to play a pivotal role in the regeneration of periodontal ligament and alveolar bone after periodontal surgery to treat periodontal disease. Therefore, the effect of a peptide of Example 1 on the expression of periostin which is a marker gene for a periodontal ligament differentiation, and the type 3 collagen fiber gene (COL3) was confirmed.

FIG. 3 is a result showing the effect of peptides of group 1 and group 2 prepared in Example 1 on expressions of type 3 collagen fiber gene (COL3) and periostin gene which are periodontal ligament marker genes, in human periodontal ligament cells.

As can be seen in FIG. 3, it was confirmed that the expression of the type 3 collagen fiber gene (COL 3) and the periostin gene was increased more than twice when the peptide group 1 and group 2 were treated in human periodontal ligament cells.

Table 5 shows the results of real-time PCR confirming the effect of the peptide groups of group 1 and group 2 on periostin mRNA expression and shows a relative mRNA expression level compared to the control. The results shown in Table 5 are the average value and standard deviation (SD) obtained by repeating the experiment three times.

**[Table 5]**

| PERIOSTIN gene expression | | |
|---|---|---|
| SEQ ID NO | Average | SD |
| 1 | 2.092 | 0.152 |
| 2 | 2.361 | 0.098 |
| 3 | 2.572 | 0.209 |
| 4 | 2.702 | 0.301 |
| 5 | 2.67 | 0.088 |
| 6 | 2.705 | 0.137 |
| 7 | 2.329 | 0.072 |
| 8 | 2.021 | 0.301 |
| 9 | 2.211 | 0.413 |
| 10 | 2.811 | 0.302 |
| 11 | 2.362 | 0.182 |
| 12 | 2.211 | 0.287 |
| 13 | 2.525 | 0.25 |
| 14 | 2.836 | 0.099 |
| 15 | 2.620 | 0.401 |
| 16 | 2.403 | 0.371 |

### Experimental Example 4: Verification of periodontium regeneration effect of peptides in periodontium damage animal model

After extraction of a 4th premolar tooth and a 2nd molar tooth from the mandibles of 4 beagle dogs, they waited for 3 months to heal, and After 3 months of healing, the 3-mural periodontal defect was induced in distal and mesial surfaces of a 1st molar tooth. Then, periodontium damage of 4x4x4 mm was formed with the buccal and distal bones remaining around the tooth root, the root alveolar bone is securely removed, tooth root planing was performed using a curette, and then an incisor groove was formed. Next, the experimental group transplanted a collagen sponge containing 50 µg of the peptide (SEQ ID NO: 16) of Example 1 to the defect region, and after the defect, a negative control (NC) without any treatment and control (NC+Plug) transplanted by placing only a collagen sponge on the inner bone wall of the bone defect was sacrificed 3 months later for histological evaluation.

FIG. 4 is a photograph of tissue stained with hematoxylin/eosin after 3 months of treatment with the peptide (SEQ ID NO: 16) of Example 1 at periodontium damage region, and shows the results of confirming an effect of a peptide on alveolar bone regeneration.

As shown in FIG. 4, it was confirmed that alveolar bone was regenerated in most of the damage periodontium in an experimental group compared to the negative control.

FIG. 5 the result of confirming an effect of the peptide on periodontal ligament tissue regeneration by staining with hematoxylin/eosin after 3 months of treatment with the peptide (SEQ ID NO: 16) of Example 1 at periodontium damage region.

As shown in FIG. 5, in a negative control (FIGS. 5A-D) without any treatment or a negative control (FIGS. 5E-H) transplanted with collagen sponge only, new periodontal ligament-like tissue (NPD) was not observed along a tooth root surface, and a connective tissue (CT) was observed. In addition, dentin in the alveolar region was observed to be absorbed. However, in an experimental group treated with the peptide (SEQ ID NO: 16) of group 2 (FIG. 5I-L), not only new cementum-like tissue was observed along a tooth root surface, but also new periodontal ligament-like tissue formation was also observed. New periodontal ligament was incorporated in the newly formed alveolar bone and cementum.

FIG. 6 is the result of confirming the effect of the peptide on periodontal ligament tissue regeneration by a collagen staining (Masson's trichrome stain) method after 3 months of treatment with the peptide (SEQ ID NO: 16) of Example 1 at periodontium damage region.

As shown in FIG. 6, in a negative control without any treatment (FIGS. 6A-B) or a negative control (FIGS. 6C-D) transplanted with collagen sponge only, most of the connective tissue (CT) was formed along a tooth root surface, and a new periodontal ligament-like tissue (NPD) was observed in some cases, but a periodontal ligament fiber bundle was irregularly arranged. In addition, in the negative control, it was observed that new cementum was formed along the tooth root. However, in an experimental group treated with the peptide (SEQ ID NO: 16) of group 2 (FIG. 6E-F), not only new cementum-like tissue was observed along a tooth root surface, but also new periodontal ligament-like tissue formation was also observed. A new periodontal ligament fiber bundle was vertically incorporated in the newly formed alveolar bone and cementum.

As such, it was confirmed that the peptide of the present invention has the effect of regenerating alveolar bone and forming a new cementum-like tissue and a new periodontal ligament-like tissue, and the new periodontal ligament formed by the peptide of the present invention is incorporated into the newly formed alveolar bone and cementum. Therefore, it was found that the peptide of the present invention can be used for regenerating periodontium, for preventing or treating a periodontal disease, and for preventing or treating a dislocated traumatic tooth.

In the present specification and drawings, preferred embodiments of the present invention have been disclosed, and although specific terms are used, these are only used in a general sense to easily explain the technical content of the present invention and help the understanding of the present invention. It is not intended to limit the scope. It will be apparent to those skilled in the art that other modifications based on the technical concept of the present invention can be performed in addition to the embodiments disclosed herein.

## Claims

1. A pharmaceutical composition for preventing or treating a periodontal disease or a dislocated traumatic tooth, comprising a peptide or a pharmaceutically acceptable salt thereof comprising an amino acid sequence of the following general formula 1:
K-Y-K-Q-X5-X6-X7-X8-Y-K (General formula 1)
in the general formula 1,
X5 to X7 are each independently arginine (R) or lysine (K);
X8 is asparagine (N) or serine (S).

2. The pharmaceutical composition of claim 1, wherein the peptide consists of an amino acid sequence of any one of SEQ ID NOs: 1 to 16.

3. The pharmaceutical composition of claim 1, comprising a polypeptide in which the peptide is linked repeatedly.

4. The pharmaceutical composition of claim 1, wherein the periodontal disease is a periodontium inflammatory disease.

5. The pharmaceutical composition of claim 1, wherein the periodontal disease is gingivitis or periodontitis.

6. The pharmaceutical composition of claim 1, further comprising a drug for treating the periodontal disease.

7. A composition for regenerating periodontium comprising a peptide comprising an amino acid sequence of the following general formula 1:
K-Y-K-Q-X5-X6-X7-X8-Y-K (General formula 1)
in the general formula 1,
X5 to X7 are each independently arginine (R) or lysine (K);
X8 is asparagine (N) or serine (S).

8. The composition of claim 7, wherein the peptide consists of an amino acid sequence of any one of SEQ ID NOs: 1 to 16.

9. The composition of claim 7, which promotes regeneration of any one or more of gingiva, periodontal ligament, cementum, and alveolar bone.

10. The composition of claim 7, wherein the composition is a pharmaceutical composition.

11. The composition of claim 7, wherein the composition is a quasi-drug composition.

12. The composition of claim 7, wherein the composition is a health functional food composition.

13. A composition for promoting expression of any one or more genes of bone sialoprotein (BSP), dentin matrix protein 1 (DMP1), Cementum attachment protein (CAP), collagen type III (COL3) and Periostin, including a peptide comprising an amino acid sequence of the following general formula 1:
K-Y-K-Q-X5-X6-X7-X8-Y-K (General formula 1)
in the general formula 1,
X5 to X7 are each independently arginine (R) or lysine (K);
X8 is asparagine (N) or serine (S).

14. The composition of claim 13, wherein the peptide consists of an amino acid sequence of any one of SEQ ID NOs: 1 to 16.
